(19) Europäisches Patentamt
European Patent Office
Office européen des brevets

(11) Veröffentlichungsnummer: **0 001 979**
**B1**

(12) **EUROPÄISCHE PATENTSCHRIFT**

(45) Veröffentlichungstag der Patentschrift: **09.09.81**

(51) Int. Cl.³: **C 07 D 213/75,**
**A 01 N 43/40**

(21) Anmeldenummer: **78101093.9**

(22) Anmeldetag: **06.10.78**

(54) **N-(2-Chlor-4-pyridyl)-N'-phenylharnstoffe, Verfahren zu deren Herstellung sowie diese enthaltende Pflanzenwachstumsregulatoren.**

(30) Priorität: **08.10.77 JP 121285/77**

(43) Veröffentlichungstag der Anmeldung:
**30.05.79 Patentblatt 79/11**

(45) Bekanntmachung des Hinweises auf die Patenterteilung:
**09.09.81 Patentblatt 81/36**

(84) Benannte Vertragsstaaten:
**CH FR GB**

(56) Entgegenhaltungen:
**DE - A - 1 445 675**
**DE - A - 1 567 214**
**DE - A - 1 620 353**
**GB - A - 1 119 515**
**GB - A - 1 122 662**
**GB - A - 1 354 830**

**Chemical Abstracts vol. 76, no. 19, 8 May 1972, T. LESIAK et al "New derivatives of urea" Seite 470, Spalte 1, Abstract Nr. 113031y**

(73) Patentinhaber: **Shudo, Koichi, Prof. Dr.**
**2000-10-2-116 Kosugayacho Totsukaku**
**Yokohama (JP)**

(73) Patentinhaber: **Isogai, Yo, Prof. Dr.**
**1-1-2-609, Kamiyoga Setagayaku**
**Tokyo (JP)**

(73) Patentinhaber: **Okamoto, Toshihiko, Prof. Dr.**
**1-7-21, Shinoharakita Kohokuku**
**Yokohama (JP)**

(73) Patentinhaber: **Sato, Susumu**
**20-7, Kamiyamacho Shibuya-ku**
**Tokyo (JP)**

(72) Erfinder: **Okamoto, Toshihiko, Prof. Dr.**
**1-7-21, Shinoharakita Kohokuku**
**Yokohama (JP)**
Erfinder: **Isogai, Yo, Prof. Dr.**
**1-1-2-609, Kamiyoga Setagaya**
**Tokyo (JP)**
Erfinder: **Shudo, Kiochi, Prof. Dr.**
**2000-10-2-116, Kosugayacho Totsukaku**
**Yokohama (JP)**
Erfinder: **Takahashi, Soshiro**
**A-415, 904-10 Oaza Kamiokubo Urawashi**
**Saitama (JP)**

(74) Vertreter: **Wuesthoff, Franz, Dr.-Ing. et al,**
**Patentanwälte Wuesthoff -v. Pechmann-Behrens-Goetz Schweigerstrasse 2**
**D-8000 München 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 001 979 B1**

Courier Press, Leamington Spa, England.

# N-(2-Chlor-4-pyridyl)-N'-phenylharnstoffe, Verfahren zu deren Herstellung sowie diese enthaltende Pflanzenwachstumsregulatoren

Die Erfindung betrifft neue N - (2 - Chlor - 4 - pyridyl) - N' - phenylharnstoffe, ein Verfahren zu deren Herstellung sowie Pflanzenwachstumsregulatoren, die als Wirkstoff diese Verbindungen enthalten.

Die Regulierung des Pflanzenwachstums mit Pflanzenhormonen ist von immer größerer Bedeutung im Bereich der Landwirtschaft und Gärtnerei. Als Pflanzenwachstumsregulatoren sind bereits 6-Benzyladenin, Kinetin und dgl. bekannt.

In der GB—PS 1 122 662 ist angegeben, daß einige Harnstoffderivate zum Schutz von Pflanzenmaterialien verwendet werden können. Außerdem ist in Proc. Soc. (London) Bd. B—165 (Seite 245) 1966 beschrieben, daß unsubstituierte N - Phenyl - N' - pyridylharnstoffe eine Wirkung zur Entwicklung einer Knospe aufweisen, während sie mäßige Wirkung als Zellteilungsinitiatoren haben.

Aus der DE—OS 15 67 214 und der GB—PS 1 119 515 ist bekannt, daß N - Pyridyl - N',N' - dialkylharnstoffe als Pflanzenwachstumsregulatoren geeignet sind. Diese Verbindungen werden jedoch tatsächlich nur als Herbizide angewandt. Als Pflanzenwachstumsregulatoren im engeren Sinne, d.h. zur Beschleunigung der Zellteilung und Zelldifferenzierung besitzen sie nur eine sehr geringe Wirksamkeit.

In der DE—OS 14 45 675 sind verschiedene Pyridylharnstoffderivate angegeben, die pharmakologische Wirksamkeit besitzen.

Pflanzenwachstumsregulatoren im Sinne der Beschreibung sind Mittel, die pflanzenbiologische Wirkungen aufweisen, wobei mit einer geringen Menge des Mittels nicht nur das Wachstum der Pflanzen gefördert oder gehemmt bzw. reguliert werden kann sondern auch, wenn gewünscht, Blumen oder Früchte erhalten, Früchte ohne Kerne erlangt, der Samen über längere Zeit im gleichen Stadium erhalten werden oder nach Wunsch weiterwachsen kann (d.h. Verhinderung des Absterbens bzw. Verhinderung des Wachstums).

Die erfindungsgemäßen Wirkstoffe als Pflanzenwachstumsregulatoren sind N - (2 - Chlor - 4 - pyridyl) - N' - phenylharnstoffe der allgemeinen Formel (I):

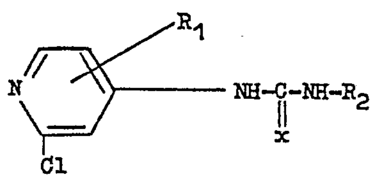

(I)

worin $R_1$ ein Wasserstoffatom oder eine niedere Alkylgruppe, $R_2$ eine, gegebenenfalls durch eine niedere Alkyl- oder Alkoxygruppe, eine Hydroxygruppe oder ein Halogenatom substituierte, Phenylgruppe und X ein Sauerstoff- oder Schwefelstoffatom bedeutet.

Die N - (2 - Chlor - 4 - pyridyl) - N' - phenylharnstoffe werden erfindungsgemäß leicht und mit guter Ausbeute dadurch hergestellt, daß man

a) ein entsprechendes 2 - Chlor - 4 - amino - pyridin mit einem entsprechenden Phenylisocyanat oder -thioisocyanat umsetzt,

b) eine entsprechendes 2 - Chlor - 4 - pyridylisocyanat oder -thioisocyanat mit Anilin oder einem entsprechenden Anilinderivat umsetzt oder

c) ein entsprechendes 2 - Chlor - isonikotinoylazid mit Anilin oder einem entsprechenden Anilinderivat umsetzt.

Zur Durchführung der Umsetzung werden die Reaktionskomponenten in einer ungefähr äquimolaren Menge in einem entsprechenden Lösungsmittel zur Reaktion gebracht, obwohl die Verwendung eines Überschusses an einer Reaktionskomponente vorteilhaft ist.

Als Reaktionslösungsmittel kommen in Frage: Benzol, Touol, Xylol, Aceton, Methyläthylketon, Dimethylformamid, Dimethylacetamid, Dimethylsulfoxyd, Chloroform, Dichlormethan, Tetrachlorkohlenstoff, Pyridin und Triäthylamid.

Ein Überschuß an Anilin wirkt gleichzeitig als Lösungsmittel. Die Umsetzung wird vorteilhaft bei einer Temperatur zwischen —5 bis 150°C durchgeführt; die Rückflußtemperatur des Reaktionsmediums ist jedoch die günstigste. Eine Reaktionsdauer von 0,5 bis 5 Stunden ist zur Erzielung einer guten Ausbeute ausreichend. Zur Herstellung des Thioharnstoffderivates muß die niedrigste Reaktionstemperatur etwas gesteigert werden und zwar im Temperaturbereich von 20 bis 150°C, weil die Reaktionsaktivität der Reaktionskomponente etwas schwächer als bei einem entsprechenden Harnstoffderivat ist.

Als typische Verbindungen kommen in Frage: N - (2 - Chlor - 4 - pyridyl) - N' - phenylharnstoff, N - (2 - Chlor - 4 - pyridyl) - N' - (2 - chlorphenyl)harnstoff, N - (2 - Chlor - 4 - pyridyl) - N' - (3 - chlorphenyl)harnstoff, N - (2 - Chlor - 4 - pyridyl) - N' - (4 - chlorphenyl)harnstoff, N - (2 - Chlor - 4 - pyridyl) - N' - (2 - methylphenyl)harnstoff, N - (2 - Chlor - 4 - pyridyl) - N' - (methylphenyl)harnstoff, N - (2 - Chlor - 4 - pyridyl) - N' - (4 - methylphenyl)harnstoff, N - (2 -

Chlor - 4 - pyridyl) - N' - (2,5 dichlorphenyl) - harnstoff, N - (2 - Chlor - 4 - pyridyl) - N' - (2 - fluorphenyl)harnstoff, N - (2 - Chlor - 4 - pyridyl) - N' - (4 - n - propylphenyl)harnstoff, N - (2 - Chlor - 4 - pyridyl) - N' - (2 - äthoxyphenyl) - harnstoff, N - (2 - Chlor - 4 - pyridyl) - N' - phenylthioharnstoff.

Die erfindungsgemäßen N - (2 - Chlor - 4 - pyridyl) - N' - phenylharnstoffe weisen eine starke und breite Aktivität als Pflanzenwachstumsregulatoren auf, besonders bei der Zellteilung, Zelldifferenzierung, Zellvergrößerung, bei der Befruchtung bzw. Verhütung des Fruchten satzes, bei der Haltung der Blume, Wachstumsförderung, Vergrößerung sowie Verzögerung des Alterungsprozesses.

Es ergibt sich jedoch noch eine weitere Anwendungsmöglichkeit, indem diese Verbindungen mit höherer Konzentration angewendet werden und damit spezielle Pflanzensorten vernichten können.

Die Wirkungen der erfindungsgemäßen Verbindungen sind bei einer niedrigen Konzentration von einem Zehntel ebenso stark wie 6-Benzyladenin und Kinetin, die bisher als die stärksten Wirkstoffe für Planzenwachstumsregulatoren angenommen wurden- und hundertfach stärker im Vergleich zu 4 - Pyridyl - phenyl - harnstoff, welcher eine gleiche Grundstruktur aufweist und kein Chloratom an der 2-Stellung des Pyrindinringes hat.

Es ist überraschend, daß lediglich die Gegenwart eines Chloratoms oder eines Äquivalents an der 2-Stellung des Pyriddinringes diese Wirkungssteigerung mit sich bringt.

Im einzelnen betragt die optimale Konzentration, die die maximale Kallusausbeute in einem Kallusvermehrungstest auf der Tabakpflanze erreicht 0,01 ppm bei 6-Benzyladenin und 0,1 ppm bei 4 - Pyridyl - phenyl - harnstoff, während sie bei den erfindungsgemäßen Verbindungen zwischen 0,0005 und 0,001 ppm (0,0001 bis 100 ppm) liegt.

Hinsichtlich der Effekte für Zellteilung ist die optimale Konzentration von 6-Benzyladenin 10 ppm, während bei den erfindungsgemäßen Verbindungen mit einer Konzentration von 1 ppm oder darunter eine erheblich größere Sproßbildung beobachtet wird.

Die erfindungsgemäßen N - (2 - Chlor - 4 - pyridyl) - N' - phenylharnstoffe wirken sich nicht nur zur erheblichen Gewichtsvermehrung von Kallus, sondern auch auf das Mark- und Blattgewebe sowie auf wachsende Pflanzen aus.

Die erfindungsgemäßen Pflanzenwachstumsregulatoren können auf eine große Anzahl verschiedener Pflanzen, bevorzugt jedoch Leguminosae, Solanaceae, Umbelliferae, Gramineae, Cucurbitaceae, Vitaceae und z.B. zur Förderung der Befruchtung von Melonen wie z.B. Wassermelonen, zur verhütung des Blütenabfalls der Weinterauben, zur Förderung des Wachstums der Gemüse und zur Vergrößerung der Tabakblätter angewendet werden. Zur Anwendung auf das Pflanzengewebe können die N - (2 - Chlor - 4 - pyridyl) - N' - phenylharnstoffe als solche dem Boden zugegeben werden, während sie zur Anwendung für wachsende Pflanzen auf die Blattoberfläche, den Stamm oder gegebenenfalls auf den Boden in einer üblichen Anwendungsform wie z.B. als wässrige Lösung oder andere Flüssigkeit, Suspension, Emulsion, Pulver und dgl. gespritzt werden können. Weitere Pflanzenwachstumsregulatoren, z.B. Düngemittel, Streckmittel und dgl. können den erfindungsgemäßen Wirkstoffen zugesetzt werden.

Die Verbindung wird in der nachstehend angegebenen Menge verwendet: Beim unmittelbaren Spritzen auf Pflanzen ist üblich: 100 bis 1000 l/ha als Lösung mit einer Konzentration von 0,0001 bis 10 000 ppm, bevorzugt von 0,01 bis 10 000 ppm. Bei einer Applikation in den Boden muß eine 5 bis 100 mal größere Menge als die oben genannte verwendet werden. Es muß wohl nicht häher erwähnt werden, daß die zu verwendende Menge schwankt, entsprechend der Pflanzenart und dem Applikationszeil, z.B.

| | |
|---|---|
| zur Wachstumbeschleunigung und vermehrten Befruchtung | 0,01—1 000 ppm |
| zur Wachstumsbeschleunigung von Kallus | 0,0001—100 ppm |
| zur Beschleunigung der Fruchtreifung und des Blütenabfalles | 0,1—10 000 ppm |
| zur Wachstumshemmung und Unkrautvernichtung | 10—> 10 000 ppm |

Nachstehend werden Beispiele der Zusammensetzung angegeben.

*Rezept 1 SPRITZPULVER*

1% N-(2-Chlor-4-pyridyl)-N'-phenyl-harnstoff
2% Polyoxyäthylenalkylaryläther
2% β-Naphthalinsulfonsäurenatrium-Formalin-Kondensat und
95% Ton

werden gemischt und gemahlen. Das Gemisch wird bei Verwendung mit Wasser verdünnt.

*Rezept 2 EMULSION*

1% N-(2-Chlor-4-pyridyl)-N'-(3-chlorphenyl)-harnstoff

4,5% Polyoxyäthylenalkylaryläther
0,5% Alkylarylsulfonat
74% Xylol und
20% Isophoron

werden gemischt und in Wasser emulgiert.

*Rezept 3 LÖSUNG*

1% N-(2-Chlor-4-pyridyl)-N'-(2-chlorphenyl)-harnstoff
5% Polyoxyäthylensorbitanmonoglykolat
94% DMF

*Rezept 4 GRANULAT*

5 Teile N-(2-Chlor-4-pyridyl)-N'-(3-methylphenyl)-harnstoff
15 Teile Bentonit
47,5 Teile Talkum
30 Teile Ton
2 Teile Natrium-ligninsulfonat und
0,5 Teile Natrium-dodecylbenzolsulfonat

werden gleichmäßig gemischt und gemahlen. Das so erhaltene Gemisch wird mit 25 Teilen Wasser gemischt, durch einen Extrusionsgranulator granuliert und das so erhaltene Granulat nach dem Trocknen gesiebt.

*Rezept 5 SPRITZPULVER*

5% N-(2-Chlor-4-pyridyl)-N'-(3-methylphenyl)-harnstoff
3% Polyoxyäthylenalkylaryläther
3% β-Naphthalinsulfonsäurenatrium-Formalin-Kondensat
89% Ton

*Rezept 6 EMULSION*

5% N-(2-Chlor-4-pyridyl)-N'-(3-chlorphenyl)-harnstoff
4,5% Polyoxyäthylalkylaryläther
0,5% Alkylarylsulfonat
70% Xylol
20% Isophoron

*Rezept 7 LÖSUNG*

100 ppm N-(2-Chlor-4-pyridyl)-N'-(3-chlorphenyl)-harnstoff
20% Aceton
80% Wasser

Die Erfindung wird durch die nachstehenden Herstellungsbeispiele näher erläutert.

Beispiel 1

*Herstellung von N-(2-Chlor-4-pyridyl)-N-phenyl-harnstoff*

a) In 10 ml getrocknetem Aceton werden 257 mg (2 mmol) 2 - Chlor - 4 - aminopyridin gelöst, 238 mg (2 mmol) Phenylisocyanat zugesetzt und bei Raumtemperatur 8 Stunden lang gerührt. Das Lösungsmittel wird unter vermindertem Druck abdestilliert. Der Rückstand wird auf Aluminiumoxid chromatographiert, mit Chloroform entwickelt, die Eluierfranktion des Endproduktes gesammelt und dann das Lösungsmittel unter vermindertem Druck abdestilliert. Der entstehende Rückstand wird aus Aceton/Äther umkristallisiert, um 364 mg N - (2 - Chlor - 4 - pyridyl) - N' - phenyl - harnstoff zu erhalten:

Ausbeute: 73,5% Schmelzpunkt 173 bis 174°C

Elementaranalyse für $C_{12}H_{10}ClN_3O$

| | C | H | N |
|---|---|---|---|
| Ber | 58,19% | 4,07% | 16,96% |
| Gef. | 58,27% | 4,15% | 16,93% |

b) Zu einer Lösung von 365 mg (2 mmol) 2-Chlorisonicotinoylazid in 10 ml getrocknetem Benzol werden 186 mg (2 mmol) Anilin zugesetzt und 3 Stunden lang unter Rückfluß erhitzt. Nach Abkühlen wird das Lösungsmittel unter vermindertem Druck abdestilliert. Der Rückstand wird auf Aluminiumoxid chromatographiert, mit Chloroform entwickelt, die Eluierfranktion des Endproduktes gesammelt und dann das Lösungsmittel unter vermindertem Druck abdestiliert. Der entstehende Rückstand wird aus Aceton/Äther umkristallisiert um 453 mg N - (2 - Chlor - 4 - pyridyl) - N' - phenylharnstoff zu erhalten. erhalten.

Ausbeute: 91,4%

Die nachstehenden Verbindungen werden wie oben beschrieben hergestellt.

| Verbindung | Schmelzpunkt (°C) |
|---|---|
| N-(2-Chlor-4-pyridyl)-N'-(2-chlorphenyl)-harnstoff | 183 |
| N-(2-Chlor-4-pyridyl)-N'-(3-chlorphenyl)-harnstoff | 198 bis 199 |
| N-(2-Chlor-4-pyridyl)-N'-(4-chlorphenyl)-harnstoff | 201 bis 201,5 |
| N-(2-Chlor-4-pyridyl)-N'-(2-methylphenyl)-harnstoff | 184 bis 185 |
| N-(2-Chlor-4-pyridyl)-N'-(3-methylphenyl)-harnstoff | 93 bis 95 |
| N-(2-Chlor-4-pyridyl)-N'-(4-methylphenyl)-harnstoff | 185,5 bis 190 |
| N-(2-Chlor-4-pyridyl)-N'-(2,5-dichlorphenyl)-harnstoff | 215 bis 216 |
| N-(2-Chlor-4-pyridyl)-N'-(2-fluorphenyl)-harnstoff | 186 bis 187 |
| N-(2-Chlor-4-pyridyl)-N'-(2-n-propylphenyl)-harnstoff | 156 bis 157 |
| N-(2-Chlor-4-pyridyl)-N'-(2-äthoxyphenyl)-harnstoff | 96 bis 99 |
| N-(2-Chlor-4-pyridyl)-N'-phenylthioharnstoff | 141 bis 142 |

Beispiel 2

*Vermehrung von Tabakkallus unter Verwendung des N-(2-Chlor-4-pyridyl)-N'-phenylharnstoffesylharn-*

Der Tabakkallus wird bei einer Temperatur von ungefähr 26°C 30 Tage lang im Murashige-Skoog-Nährboden (Physiol Plant 15,473 (1962)), welcher 0,0001 bis 0,1 ppm N - (2 - Chlor - 4 - pyridyl) - N' - phenylharnstoff und 2 ppm Indulessigsäure als Auxin enthält, kultiviert, Das endgültige Rohgewicht wird in Tabelle 1 zusammengefaßt. Als Kontrolle wird ein Kultivieren des Tabakkallus unter gleichen Bedingungen, mit Murashige-Skoog-Nährboden, welcher jedoch nur ein Auxin enthält, durchgeführt. Ausserdem wird in der Tabelle zum Vergleich das Rohgewicht des Kallus unter Verwendung eines Nährbodens, welcher die optimale Konzentation von Benzyladenin enthält, gezeigt. Die in der Tabelle gezeigten Gewichte sind jeweils Mittelwerte von 6 Proben.

TABELLE 1

| *Konzentration des Wirkstoffes (ppm)* | Rohgewicht (mg) |
|---|---|
| N-(2-Chlor-4-pyridyl)-N'-phenylharnstoff 0,0001 | 782 |
| N-(2-Chlor-4-pyridyl)-N'-phenylharnstoff 0,001 | 4 991 |
| N-(2-Chlor-4-pyridyl)-N'-phenylharnstoff 0,01 | 1 235 |
| N-(2-Chlor-4-pyridyl)-N'-phenylharnstoff 0,1 | 519 |
| Benzyladenin 0,01 | 5 150 |
| Kontrolle | 153 |

Beispiel 3

*Vermehrung von Tabakkallus mit N-(2-Chlor-4-pyridyl)-N'-(3-methylphenyl)-harnstoff*

Durch Kultivierung des Tabakkallus, unter Verwendung des obigen Harnstoffes, wie in Beispiel 2 beschrieben, entstehen die in Tabelle 2 gezeigten Ergebnisse.

TABELLE 2

| *Konzentration des Wirkstoffes (ppm)* | Rohgewicht (mg) |
|---|---|
| 0,001 | 1 784 |
| 0,01 | 6 760 |
| 0,1 | 4 945 |
| 1 | 1 508 |
| Kontrolle | 153 |

Vergleichbare Ergebnisse entstehen, wenn N - (2 - Chlor - 4 - pyridyl) - N' - (3 - chlorphenyl) - harnstoff verwendet wird.

Beispiel 4

*Sprossbildung aus dem Markgewebe mit N-(2-Chlor-4-pyridyl)-N'-phenylharnstoff*

Mehrere Schnitte des Tabakmarkes werden in einem Murashige-Skoog-Nährboden, welcher 0,01 bis 10 ppm N - (2 - Chlor - 4 - pyridyl) - N' - phenylharnstoff enthält, geimpft und bei einer Temperatur von ungefähr 26°C 30 Tage lang kultiviert. Danach werden die Markschnitte, aus denen Sprosse gebildet wurden, gezählt.

Zum Vergleich wird Benzyladenin ebenfalls untersucht.

Die Ergebnisse werden in Tabelle 3 zusammengefaßt.

TABELLE 3

Sprossbildung aus dem Markschnitt

| Konzentration des Wirkstoffes (ppm) | Sprossbildung +) | |
|---|---|---|
| | Benzyladenin | N-(2-Chlor-4-pyridyl)-N'-phenylharnstoff |
| 10 | 1/24 | 10/24 |
| 1 | 12/24 | 13/24 |
| 0,1 | 2/24 | 10/24 |
| 0,01 | 0/24 | 0/24 |

+) Anzahl der sich in den Markschnitten gebildeten Sprosse/Anzahl der geimpften Markschnitte.

Aus einem Markschnitt werden 1 bis 6 Sprosse gebildet.

Beispiel 5

*Sprossbildung aus Kallus mit N-(2-Chlor-4-pyridyl)-N'-(2-methylphenyl)-harnstoff*

Einige Tabakkallus werden in einem Murashige-Skoog-Nährboden, welcher 0,01 bis 10 ppm N - (2 - Chlor - 4 - pyridyl) - N' - (2 - methyl) - harnstoff enthält, geimpft und bei Raumtemperatur 30 Tage lang kultiviert. Die Anzahl der sich in den Kallusschnitten gebildeten Sprosse wird gezählt. Zum Vergleich wird Benzyladenin ebenfalls untersucht.

Die Ergebnisse werden in Tabelle 4 zusammengefaßt.

TABELLE 4

*Sprossbildung aus Tabakkallus*

| Konzentration des Wirkstoffes (ppm) | Sprossbildung [+] | |
|---|---|---|
| | Benzyladenin | N-(2-Chlor-4-pyridyl)-N'-(2-methylphenyl)-harnstoff |
| 10 | 12/12[a] | 10/12[a] |
| 1 | 12/12[a] | 9/12[a] |
| 0,1 | 2/12[b] | 1/12[b] |
| 0,01 | 0/12 | 0/12 |

[+] Anzahl der sich in dem Kallus gebildeten Sprosse/Anzahl der geimpften Kallus.

a) 50 bis 70 Sprosse (Größe jedes Sprosses: 1 bis 3 cm)
b) 1 bis 10 Sprosse (Größe jedes Sprosses: 0,5 bis 1 cm)

Beispiel 6

*Sprossbildung aus Kallus mit N-(2- Chlor-4-pyridyl)-N'-phenylharnstoff*

Der Nährboden, welcher 0,0001 bis 1 ppm N - (2 - Chlor - 4 - pyridyl) - N' - phenyl - harnstoff enthält, wird hergestellt. Die Kultivierung wird wie in Beispiel 5 beschrieben vorgenommen. Die Ergebnisse werden in Tabelle 5 zusammengefaßt.

TABELLE 5

*Sprossbildung aus Kallus*

| Konzentration des Wirkstoffes (ppm) | Sprossbildung [+] | |
|---|---|---|
| | Benzyladenin | N-(2-Chlor-4-pyridyl)-N'-phenylharnstoff |
| 10 | 12/12[a] | — |
| 1 | 12/12[b] | 12/12[a] |
| 0,1 | 2/12[b] | 12/12[a] |
| 0,01 | 0/12 | 12/12[a] |
| 0,001 | — | 2/12[b] |
| 0,0001 | — | 0/12 |

[+] Anzahl der sich in den Kallus gebildeten Sprosse/Anzahl der geimpften Kallus.

a) 50 bis 70 Sprosse (Größe jedes Sprosses 1 bis 3 cm)
b) 1 bis 10 Sprosse (Größe jedes Sprosses 0,5 bis 1 cm)

Beispiel 7

*Vergrößerung einer Art Raps mit N-(2-Chlor-4-pyridyl)-N'-phenyl-harnstoff*

Es wird eine wässrige Lösung von 10 ppm, 1 ppm und 0,1 ppm N - (2 - Chlor - 4 - pyridyl) - N' - phenylharnstoff hergestellt. In eine Schale werden jeweils 50 ml Lösung eingegossen und 10 Blattschnitte, die mit Hilfe eines Korkbohrers mit einem Durchmesser von 10 mm ausgeschnitten werden,

1) mit der Rückseite des Blattes auf die Lösung oder
2) mit der Oberseite des Blattes auf die Lösung gelegt.

Die Lösung wird bei Raumtemperatur 8 Tage lang stehengelassen, dann werden der Durchmesser und das Gewicht jedes Blattschnittes bestimmt. Alle Werte sind Mittelwerte von 10 Blattschnittproben. Als Vergleichslösung wird reines Wasser verwendet und analog verfahren. Die Ergebnisse werden in Tabelle 6 zusammengefaßt.

TABELLE 6

1)

| Konzentration (ppm) | Durchmesser (mm) | Gewicht (mg) |
|---|---|---|
| 10 | 13,7 | 30,5 |
| 1 | 13,5 | 32,1 |
| 0,1 | 14,2 | 33,3 |
| Vergleichslösung | 12,1 | 21,7 |

2)

| Konzentration (ppm) | Durchmesser (mm) | Gewicht (mg) |
|---|---|---|
| 10 | 13,2 | 26,8 |
| 1 | 14,2 | 31,1 |
| 0,1 | 13,9 | 28,1 |
| Vergleichslösung | 11,9 | 21,0 |

Beispiel 8

*Untersuchung über eine vermehrte Befruchtung der Wassermelone*

Die im Freien gezüchteten Wassermelonen (Sorte: Yamato) werden der künstlichen Befruchtung in der Blütezeit unterworfen. Dann werden auf die Fruchtstiele bestimmte Konzentrationen der in Tabelle 7 angegebenen, verschiedenen Lösungen aufgetragen bzw. sie werden damit besprüht. Das Ergebnis der vermehrten Befruchtung (in %) wird 2 Wochen nach der Behandlung errechnet.

TABELLE 7

| Methode der Behandlung | Mittel | Konzentration (ppm) | Befruchtung (%) |
|---|---|---|---|
| Auftragen | N-(2-Chlor-4-pyridyl)-N-phenyl-harnstoff | 1 000 | 100 |
| | 6-(N-Benzyl)-aminopurin in 3 %iger Lösung | 1 000 | 65 |
| | | 10 000 | 100 |
| | ohne Behandlung | – | 43 |
| Besprühen | N-(2-Chlor-4-pyridyl)-N-phenyl-harnstoff | 1 000 | 90 |
| | | 500 | 87 |
| | | 100 | 82 |
| | 6-(N-Benzyl)-aminopurin in 3 %iger Lösung | 10 000 | 83 |
| | ohne Behandlung | – | 35 |

Beispiel 9

*Hemmung der Pflanzengröße, Vergrößerung des Blattes und Erhaltung der grünen Frabe der Tabakpflanzen*

Die Tabaksamen (Sorte: bright yellow) werden in einer unglasierten Tontopf mit einem Durchmesser von ca. 13 cm gesät und in einem Glashaus gezüchtet. Die Blattoberflächen der Pflanzen, die 6 bis 8 Blätter aufweisen, werden (15 ml/Topf) mit bestimmten Konzentrationen der in Tabelle 8 angegebenen Verbindungen mit einem Sprühgerät gleichmäßig besprüht. Eine Sektion der Anpflanzung wird einmal behandelt, die andere Sektion wird dreimal hintereinander und zwar jeden 13. Tag, behandelt.

Wenn die mittleren Blätter gelb werden (35 Tage nach der ersten Behandlung mit dem Mittel) werden die Pflanzengröße, das Rohgewicht des Stengels und der Blätter und die durchschnittliche Größe der unteren 5 Blätter gemessen und das Verhältnis zu den nicht behandelten Pflanzen errechnet. Die Untersuchungssektion wird in 3 Teile geteilt und die Ergebnisse im Durchschnitt gezeigt. Die Ergebnisse sind in Tabelle 8 zusammengefaßt.

TABELLE 8

| Verbindung | Konzentration (ppm) | Häufigkeit des Bestreuens /Besprühens | Verhältnis zur nicht behandelten Sektion | | |
|---|---|---|---|---|---|
| | | | Pflanzengröße | Rohgewicht | Größe der Blätter |
| N-(2-Chlor-4-pyridyl)-N-phenyl-harnstoff aus 5 %ig. Spritzpulver | 500 | 1 | 74 | 171 | 158 |
| | | 3 | 42 | 169 | 145 |
| | 100 | 1 | 78 | 188 | 175 |
| | | 3 | 72 | 206 | 185 |
| | 20 | 1 | 102 | 178 | 172 |
| | | 3 | 98 | 160 | 152 |
| 6-(Benzyl)-aminopurin aus 3 %iger Lösung | 500 | 1 | 99 | 151 | 118 |
| | | 3 | 85 | 138 | 111 |
| | 100 | 1 | 98 | 118 | 141 |
| | | 3 | 90 | 115 | 124 |
| | 20 | 1 | 106 | 109 | 120 |
| | | 3 | 98 | 113 | 121 |
| ohne Behandlung | | 100 (74,6 cm) | 100 (126,6 g /Pflanze) | 100 (277,9 $cm^2$/Blatt) | – |

Beispiel 10

*Untersuchung zur Verhinderung des vorzeitigen Blütenabfalls des Weinstocks und Beschleunigung der Befruchtung*

Die kernlosen, achtjährigen Pflanzen von Delaware-Weintrauben, die in einem nicht erwärmten Haus gezüchtet und mit Gebberellin behandelt wurden, werden auf Verhinderung des vorzeitigen Blütenabfalls der Weintrauben durch N - (2 - Chlor - 4 - pyridyl) - N' - (2 - methylphenyl) - harnstoff geprüft. Nachdem ein Spross an der Pflanze 8 bis 8,5 neue Blätter entwickelt hat, d.h. 3 bis 5 Tage bevor er zur ersten Gibberellinbehandlung geeignet ist, um ihn kernlos zu machen, werden die Blütenbüschel mit einer wässrigen Lösung von 100 ppm Gibberellin und 200, 100, 50 oder 25 ppm N - (2 - Chlor - 4 - pyridyl) - N' - (2 - methylphenyl) - harnstoff oder 200 bzw. 100 ppm 6 - (n - Benzyl) - aminopurin (BA) imprägniert (14. April). Am 4, Mai (10 Tage nach der vollen Blüte) wurden alle Blüten mit weiteren 100 ppm der Gibberellinlösung imprägniert und dann in der üblichen Zuchtmethode bis zur Erntezeit gezüchtet. Am 29. Juni wurden die Weintrauben geerntet und Gewicht, Länge, Dichtigkeit der Beeren, Anzahl der Beeren in einer Traube, sowie Durchmesser und Gewicht des Fruchtstieles untersucht. Die Ergebnisse werden mit den nur mit Gibberellin behandelten Trauben verglichen. Die Untersuchungssektion, bestehend aus jeweils 2 Sprossen, wird jeweils in 3 Teile geteilt. Die Durchschnittsergebnisse von 15 Weintrauben werden in Tabelle 9 zusammengefaßt.

TABELLE 9

| Verbindung + 100 ppm Gibberellin | Konzentr. | Traube Gewicht | Traube Länge | Beere Dichtigkeit | Beere Anzahl | Kernlosigkeit (%) | Fruchtstiel Durchmesser (mm) | Fruchtstiel Gewicht (g) | Fruchtstiel Härte |
|---|---|---|---|---|---|---|---|---|---|
| Rezept 3 | 200 | 195,6 (190) | 13,7 (91) | 14,3 (210) | 136,6 (194) | 100 | 5,08 (213) | 5,96 (248) | hart |
| | 100 | 196,3 (190) | 14,5 (97) | 13,5 (199) | 136,3 (194) | 100 | 4,60 (192) | 5,47 (228) | etwas hart bis hart |
| | 50 | 189,8 (184) | 14,1 (94) | 13,5 (199) | 129,6 (184) | 100 | 4,62 (193) | 4,71 (196) | etwas hart bis hart |
| | 25 | 180,8 (175) | 15,0 (100) | 12,1 (178) | 142,3 (202) | 100 | 3,82 (160) | 4,71 (196) | etwas hart bis hart |
| 3 %ige Lösung von BA | 200 | 136,6 (132) | 14,8 (99) | 9,2 (135) | 93,3 (133) | 100 | 3,32 (139) | 2,75 (115) | normal |
| | 100 | 125,3 (122) | 15,5 (103) | 8,1 (119) | 73,9 (105) | 100 | 3,02 (126) | 2,46 (103) | etwas weich bis normal |
| nur Gibberellin | (100) | 103,1 (100 %) | 15,0 (100 %) | 6,8 (100 %) | 70,3 (100 %) | 100 | 2,39 (100 %) | 2,40 (100 %) | etwas weich |

Die in Klammer gesetzten Werte zeigen jeweils das Verhältnis zum Ergebnis der nur mit Gibberellin behandelten Trauben.

Beispiel 11

*Herbizideffekt*

In einen Topf mit einem Durchmesser von 30 cm wird Boden eingefüllt, der die Wurzeln von Alisma canaliculatum (Froschlöffel) und Scirpus juncoides (Simse) zu gleichen Teilen enthält. In den Topf werden 50 Samen von Echinochloa Crus-galli (Hühnerfennich), Cyperus difformis und Monochoria vaginalis gesät, dazu werden je Topf 5 Reispflanzen (Orysa sativa) im 2-Blätterstadium eingepflanzt, dann in Wasser getaucht, so daß das Wasser 3 cm über dem Rand des Topfes steht. Wenn der Hühnerfennich das erste Blattstadium erreicht, wird eine bestimmte Menge von einem gemäß Rezept 1 hergestellten Herbizid gleichmäßig auf das Wasser verteilt. 14 Tage später werden die Unkrautverhinderungseffekte untersucht.

Die Ergebnisse werden gemäß der folgenden Liste gezeigt:

Index:

5 : Vollkommene Unkrautverhinderung

4 : über 80%

3 : über 60%

2 : über 40%

1 : über 20%

0 : keine Effekte

| Verbindung | Wirkstoff kg/ha | Hühner-fennich | Cyperus | Monochoria | Simse | Froschlöffel | Schädigung des Reises |
|---|---|---|---|---|---|---|---|
| N-(2-Chlor-4-pyridyl)-N-phenyl-harnstoff | 100 | 5 | 5 | 5 | 4 | 5 | 0 |
| | 50 | 4 | 5 | 4,5 | 3 | 4,5 | 0 |

Beispiel 12

Untersuchung der Zunahme von Blättern und neuen Zweigen bei Datura Sunguinea.

Datura Sunguinea sp. (mittlere Höhe 8 cm) werden ins Freiland gesetzt. Lösungen der gewünschten Konzentration werden in einer Menge von 15 ml je Pflanze auf die Blätter und Blattstiele der Pflanzen aufgesprüht, wenn die mittlere Höhe der Pflanzen 20 cm beträgt. 3 Wochen später werden die Pflanzen geerntet und die Höhe der Pflanzen, das Gesamtgewicht von frischen Blättern und Stielen und die neuen Zweige gemessen. In der Tabelle ist jeweils der Mittelwert von 5 Pflanzen angegeben.

| Verbindung | Konzentration (ppm) | Höhe (cm) | Gesamtgew. (g) | neue Zweige (g) |
|---|---|---|---|---|
| N-(chlor-4-pyridyl-N'-phenyl harnstoff | 20 | 80 | 450 | 80 |
| " | 100 | 75 | 500 | 100 |
| BA | 500 | 90 | 402 | 70 |
| Vergleich | | 103 | 348 | 40 |

Beispiel 13

Untersuchung der Zunahme von Blättern und neuen Zweigen bei Datura Sunguinea.

Datura Sunguina sp. (mittlere Höhe 8 cm) werden ins Freiland gepflanzt und Untersuchungen unter Verwendung von N - (2 - Chlor - 4 - pyridyl) - N' - (3 - bromphenyl) - harnstoff werden wie in Beispiel 12 durchgeführt. Die Ergebnisse sind in der folgenden Tabelle angegeben.

| Verbindung | Konzentration (ppm) | Höhe (cm) | Gesamtgewicht. (g) | neue Zweige (g) |
|---|---|---|---|---|
| N-(2-Chlor-4-pyridyl)-N'-(3-bromphenyl)-harnstoff | 60 | 81 | 450 | 78 |
| " | 300 | 74 | 490 | 98 |
| BA | 500 | 91 | 391 | 68 |
| Vergleich | | 102 | 352 | 41 |

**Patentansprüche**

1. N - (2 - Chlor - 4 - pyridyl) - N' - phenyl - harnstoffe der allgemeinen Formel (I)

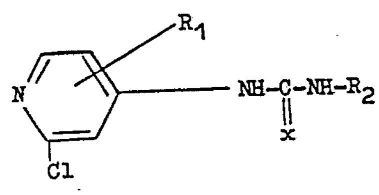

(I)

worin $R_1$ ein Wasserstoffatom oder eine niedere Alkylgruppe, $R_2$ eine, gegebenenfalls durch eine niedere Alkyl- oder Alkoxygruppe, eine Hydroxygruppe oder ein Halogenatom substituierte, Phenylgruppe und X ein Sauerstoff- oder Schwefelstoffatom bedeutet, sowie deren Säureadditionssalze.

2. N - (2 - Chlor - 4 - pyridyl) - N' - phenyl - harnstoff, nach Anspruch 1 dadurch gekennzeichnet, daß $R_1$ ein Wasserstoffatom bedeutet, sowie deren Säureadditionssalze.

3. N - (2 - Chlor - 4 - pyridyl) - N' - phenyl - harnstoffe nach Anspruch 1 dadurch gekennzeichnet, daß X ein Sauerstoffatom bedeutet, sowie deren Säureadditionssalze.

4. N - (2 - Chlor - 4 - pyridyl) - N' - phenyl - harnstoffe nach Anspruch 1 dadurch gekennzeichnet, daß jeweils $R_1$ ein Wasserstoffatom, X ein Sauerstoffatom und $R_2$ eine mit einem Halogen-

atom oder einer Methylgruppe substituierte Phenylgruppe bedeutet, sowie deren Säureadditionssalze.

5. N - (2 - Chlor - 4 - pyridyl) - N' - phenylharnstoff und dessen Säureadditionssalze.

6. N - (2 - Chlor - 4 - pyridyl) - N' - (3 - chlorphenyl) - harnstoff sowie desse Säureadditionssalze.

7. N - (2 - Chlor - 4 - pyridyl) - N' - (3 - methylphenyl) - harnstoff sowie dessen Säureadditionssalze.

8. Verfahren zur Herstellung von N - (2 - Chlor - 4 - pyridyl) - N' - phenyl harnstoffen nach Anspruch 1, dadurch gekennzeichnet, daß man

a) ein entsprechendes 2 - Chlor - 4 - amino - pyridin mit einem entsprechenden Phenylisocyanat umsetzt oder

b) ein entsprechendes 2 - Chlor - 4 - pyridylisocyanat mit einem entsprechenden Anilin umsetzt oder

c) ein entsprechendes 2 - Chlor - isonikorinoylazid mit einem entsprechenden Anilin umsetzt.

9. Pflanzenwachstumsregulatoren, dadurch gekennzeichnet, daß sie als Wirkstoff mindestens einen N - (2 - Chlor - 4 - pyridyl) - N' - phenyl - harnstoff nach Anspruch 1 enthalten.

**Revendications**

1. N - (2 - Chlor - 4 - pyridyl) - N' - phénylurées de formule générale (I)

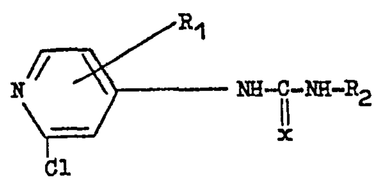

(I)

où $R_1$ représente un atome d'hydrogène ou un groupe alcoyle inférieur, $R_2$ un groupe phényle éventuellement substitué par un groupe alcoyle ou alcoxy inferieur, un groupe hydroxy ou un atome, d'halogène, et X un atome d'oxygène ou de soufre, ainsi que leurs sels d'addition acides.

2. N - (2 - chloro - 4 - pyridyl) - N' - phényl - urées selon la revendication 1, caractérisées en ce que $R_1$ représente un atome d'hydrogène, ainsi que leurs sels d'addition acides.

3. N - (2 - chloro - 4 - pyridyl) - N' - phényl - urées selon la revendication 1, caractérisées en ce que X représente un atome d'oxygène, ainsi que leurs sels d'addition acides.

4. N - (2 - chloro - 4 - pyridyl) - N' - phényl - urées selon la revendication 1, caractérisé en ce que, respectivement, $R_1$ représente un atome d'hydrogène, X un atome d'oxygène et $R_2$ un groupe phényle substitué par un atome d'halogène ou un groupe méthyle, ainsi que leurs sels d'addition acides.

5. N - (2 - chloro - 4 - pyridyl) - N' - phénylurée et ses sels d'addition acides.

6. N - (2 - chloro - 4 - pyridyl) - N' - (3 - chlorophényl est ses sels d'addition acides.

7. N - (2 - chloro - 4 - pyridyl) - N' - (3 - méthylphényl) - urée et ses d'addition acides.

8. Procédé de préparation de N - (2 - chloro - 4 - pyridyl) - N' - phényl - urées selon la revendication 1, caractérisé en ce que

a) on fait réagir une 2 - chloro - 4 - amino - pyridine correspondante avec un phénylisocyanate correspondant ou

b) on fait réagir un 2 - chloro - 4 - pyridylisocyanate correspondant avec une aniline correspondante ou

c) on fait réagir un azothydrure de 2 - chloro - isonicotinoyle correspondant avec une aniline correspondante.

9. Régulateurs de la croissance des plantes caractérisés en ce qu'ils contiennent comme matière active au moins une N - (2 - chloro - 4 - pyridyl) - N' - phényl-urée selon la revendication 1.

**Claims**

1. N - (2 - chloro - 4 - pyridyl) - N' - phenyl - ureas of the general formula (I)

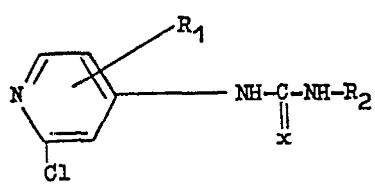

(I)

wherein $R_1$ is a hydrogen atom or a lower alkyl group, $R_2$ a phenyl group optionally substituted by a lower alkyl or alkoxy group, a hydroxy group or a halogen atom, and X is an oxygen atom or sulfur atom as well as the acid addition salts thereof.

2. N - (2 - chloro - 4 - pyridyl) - N' - phenyl - ureas as claimed in claim 1 characterized in that $R_1$ is a hydrogen atom as well as the acid addition salts thereof.

3. N - (2 - chloro - 4 - pyridyl) - N' - phenyl - ureas as claimed in claim 1 characterized in that X is an oxygen atom as well as the acid addition salts thereof.

4. N - (2 - chloro - 4 - pyridyl) - N' - phenyl - ureas as claimed in claim 1 characterized in that $R_1$ is a hydrogen atom, X an oxygen atom and $R_2$ a phenyl group substituted by a halogen atom or a methyl group as well as the acid addition salts thereof.

5. N - (2 - chloro - 4 - pyridyl) - N' - phenyl - urea and its acid addition salts.

6. N - (2 - chloro - 4 - pyridyl) - N' - (3 - chlorophenyl) - urea as well as its acid addition salts.

7. N - (2 - chloro - 4 - pyridyl) - N' - (3 - methylphenyl) - urea as well as its acid addition salts.

8. Process for producing N - (2 - chloro - 4 - pyridyl) - N' - phenylureas as claimed in claim 1 characterized in that

a) a corresponding 2 - chloro - 4 - amino - pyridin is reacted with a corresponding phenyl isocyanate or

b) a corresponding 2 - chloro - 4 - pyridyl isocyanate is reacted with a corresponding aniline or

c) a corresponding 2 - chloro - isonikotinoylazid is reacted with a corresponding aniline.

9. Plant growth regulators characterized in that as active component they contain at least one N - (2 - chloro - 4 - pyridyl) - N' - phenyl - urea as claimed in claim 1.